# EUROPEAN PATENT APPLICATION

(11) **EP 4 282 965 A1**
(43) Date of publication of application: **29.11.2023**
(21) Application number: 22774121.2
(22) Date of filing: 17.03.2022
(51) Int. Cl.: C12N 15/113, C12N 15/77, C12N 15/67, C12N 1/21, C12N 9/12, C12P 13/08, C12P 13/06, C12P 13/12, C12P 13/00, C12P 7/44, C12R 1/15

(54) **ASPARTATE KINASE GENE EXPRESSION REGULATORY SEQUENCE AND USE THEREOF**

(30) Priority: 23.03.2021 CN 202110305010
(71) Applicant: Tianjin Institute of Industrial Biotechnology, Chinese Academy of Sciences, Tianjin Airport Economic Area Tianjin 300308 (CN)
(72) Inventor: SUN, Jibin, Tianjin 300308 (CN); LIU, Jiao, Tianjin 300308 (CN); ZHENG, Ping, Tianjin 300308 (CN); SHI, Tuo, Tianjin 300308 (CN); ZHOU, Wenjuan, Tianjin 300308 (CN); MA, Yanhe, Tianjin 300308 (CN)
(74) Representative: Gille Hrabal Partnerschaftsgesellschaft mbB Patentanwälte
(86) International application number: PCT/CN2022/081527
(87) International publication number: WO 2022/199460

(57) **Abstract**

Disclosed are an aspartate kinase gene expression regulatory sequence and use thereof. By modifying positions 366-373 of a sequence represented by SEQ ID NO: 1, a polynucleotide formed by linking the sequence with a start codon GTG or TTG has transcriptional expression regulation activity, and these polynucleotides can improve the expression of the aspartate kinase encoding gene *lysC,* such that a large amount of L-lysine is accumulated.

## Description

### TECHNICAL FIELD

The present disclosure belongs to the field of molecular biology and bioengineering, and in particular relates to a polynucleotide sequence for regulating transcriptional expression of an aspartate kinase gene, and a method for producing L-lysine by utilizing the polynucleotide sequence to be transcribed and to express aspartate kinase.

### BACKGROUND

L-lysine, called lysine for short, is a basic amino acid and the most important essential amino acid in human and animal nutrition. Due to the low content of lysine in cereal foods, and its lack because of the destruction in the processing process, lysine is called the first limiting amino acid and is widely applied to the industries of medicine, health, food, animal feed and the like. Lysine is mainly produced by microbial fermentation, and at present, the main production strains include microorganisms such as *Escherichia coli*, *C. glutamicum* and the like.

The synthetic pathway of L-lysine begins with aspartic acid in many microorganisms, and includes two steps shared with methionine and threonine, followed by a nine-step enzymatic process to ultimately produce L-lysine. Therein, aspartate kinase (ASK or AK, encoded by gene *lysC*) is the first enzyme in the synthetic pathway of L-lysine initiated by aspartic acid, and its activity determines the ratio of metabolic flux flowing to the synthetic pathway of L-lysine, which is the rate-limiting step in lysine production. The activity of aspartate kinase is under complex regulation in microorganisms. In many L-lysine-producing bacteria, the activity of aspartate kinase is inhibited by the negative feedback of the end product L-lysine. At present, a large number of aspartate kinase mutants that are relieved of feedback inhibition have been reported, such as EP2374873A1. How to further improve the expression of the feedback inhibition-relieved aspartate kinase is an essential way for microorganisms to produce lysine with high yield.

### SUMMARY

In order to solve the problems existing in the prior art, it is an object of the present disclosure to provide a polynucleotide sequence that enhances the expression regulation of gene *lysC.* Through a great deal of effort, the inventors of the present disclosure have found that by modifying a partial sequence TTACTCTA (i.e., positions 366 to 373 of the polynucleotide sequence set forth in SEQ ID NO: 1) in a spacer between a ribosome binding site (hereinafter referred to as RBS) and a start codon of an artificial expression regulatory sequence (the nucleotide sequence of which is set forth in SEQ ID NO: 1), a polynucleotide sequence formed by linking the sequence to a start codon GTG or TTG has transcriptional expression regulatory activity, and such a polynucleotide sequence having transcriptional expression regulatory activity can increase the expression of the aspartate kinase-encoding gene *lysC,* and then achieve a large accumulation of L-lysine, on the basis of which the present disclosure is completed.

It is another object of the present disclosure to provide a vector comprising a polynucleotide sequence that exhibits improved promoter activity.

It is still another object of the present disclosure to provide a host cell containing an expression regulatory sequence of gene *lysC.*

It is yet another object of the present disclosure to provide a method for producing L-lysine by fermenting a host cell.

In order to achieve the objectives above, the present disclosure adopts the following technical solutions:
In a first aspect, the present disclosure provides a polynucleotide for regulating the expression of a gene, wherein the polynucleotide is:
(1) a polynucleotide sequence formed by linking a sequence resulting from substitution and/or deletion of one or more nucleotides at positions 366 to 373 in a sequence set forth in SEQ ID NO: 1 to a start codon GTG;
   or
(2) a polynucleotide sequence formed by linking a sequence resulting from substitution and/or deletion of one or more nucleotides at positions 366 to 373 in a sequence set forth in SEQ ID NO: 1 to a start codon TTG;
compared with the unmodified SEQ ID NO: 1, the polynucleotide has the activity of enhancing the expression of gene *lysC.*

Preferably, the polynucleotide has a nucleotide sequence set forth in SEQ ID NO: 2, SEQ ID NO: 3 or SEQ ID NO: 4.

In a second aspect, the present disclosure provides a vector comprising the polynucleotide described above.

Preferably, the vector is based on pK18mobsacB as a backbone, and the GenBank accession number of the pK18mobsacB is FJ437239.1.

In a third aspect, the present disclosure provides an expression cassette for regulating the expression of gene *lysC,* wherein the expression cassette is a polynucleotide formed by operably linking the polynucleotide described above to a gene *lysC* without a start codon. Preferably, the polynucleotide has a nucleotide sequence set forth in SEQ ID NO: 2, SEQ ID NO: 3 or SEQ ID NO: 4.

In a fourth aspect, the present disclosure provides a host cell comprising the polynucleotide described above or the expression cassette described above.

Preferably, the polynucleotide has a nucleotide sequence set forth in SEQ ID NO: 2, SEQ ID NO: 3 or SEQ ID NO: 4.

Optionally, the host cell is derived from *Corynebacterium, Brevibacterium, Arthrobacter, Microbacterium,* or *Escherichia*; preferably, the host cell is *C. glutamicum* or *Escherichia coli*; and more preferably, the host cell is *C. glutamicum* ATCC 13032, *C. glutamicum* ATCC 13869, or *C. glutamicum* ATCC 14067, or a derivative strain thereof.

In a fifth aspect, the present disclosure provides a method for enhancing the expression of an aspartate kinase-encoding gene *lysC,* the method is an operable linkage of the polynucleotide described above to a gene *lysC* without a start codon.

Preferably, the polynucleotide has a nucleotide sequence set forth in SEQ ID NO: 2, SEQ ID NO: 3 or SEQ ID NO: 4.

Preferably, the operable linkage is to introduce a vector containing the polynucleotide described above into a host cell, and to integrate the polynucleotide into a genome of the host cell by homologous recombination.

Further preferably, the host cell is derived from *Corynebacterium, Brevibacterium, Arthrobacter, Microbacterium,* or *Escherichia*; preferably, the host cell is *C. glutamicum* or *Escherichia coli*; and more preferably, the host cell is *C. glutamicum* ATCC 13032, *C. glutamicum* ATCC 13869, or *C. glutamicum* ATCC 14067.

Still further preferably, the host cell is *C. glutamicum* modified as follows: 1) a mutation coding sequence of T311I is introduced into an aspartate kinase-encoding gene *lysC* in *C. glutamicum* ATCC13032; 2) a core region from position 279 to position 317 of the pyruvate carboxylase gene *pyc* promoter in *C. glutamicum* is CGGGCCTTGATTGTAAGATAAGACATTTAGTATAATTAG; and 3) nucleotides from position 279 to position 317 of the diaminopimelate dehydrogenase gene *ddh* promoter in *C. glutamicum* are mutated from wild type ATGCATCTC to CCTTGTTAT.

In a sixth aspect, the present disclosure provides use of the polynucleotide in the first aspect, the vector in the second aspect, the expression cassette in the third aspect, and the host cell in the fourth aspect in the preparation of L-lysine.

In a seventh aspect, the present disclosure provides a method for producing L-lysine, the method comprises a step of culturing the host cell in the fourth aspect to produce L-lysine. Optionally, the method comprises a step of isolating L-lysine from a fermentation broth.

In an eighth aspect, the present disclosure provides use of the polynucleotide in the first aspect, the vector in the second aspect, the expression cassette in the third aspect, and the host cell in the fourth aspect in the preparation of aspartate acid family-amino acids and derivatives thereof, the aspartate-family amino acids and the derivatives thereof including L-threonine, L-isoleucine, L-homoserine, L-methionine and downstream products of L-lysine such as pentanediamine, 5-aminopentanoic acid, and glutaric acid.

### Beneficial Effects of Present Disclosure

The present disclosure provides a polynucleotide having the activity of enhancing the expression of gene *lysC,* which can be operably linked to the target gene *lysC* to remarkably improve the expression intensity of *lysC,* thus producing downstream products stably and efficiently.

The present disclosure provides a method for producing L-lysine, which can improve the expression of aspartate kinase by using the polynucleotide having gene transcriptional expression regulatory activity described above, thus producing L-lysine stably and efficiently, the yield of L-lysine can reach 1.9 times that of an initial strain.

### BRIEF DESCRIPTION OF THE DRAWINGS

FIG. 1 shows a plasmid map of pEC-XK99E-*rfp*-1;
FIG. 2 shows a plasmid map of pEC-XK99E-*rfp*-2.

### DETAILED DESCRIPTION

The technical solutions of the present disclosure will be further illustrated in detail with reference to the following specific examples. It will be understood that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products or can be prepared using known methods. Where specific conditions are not indicated in experimental methods in the following examples, conventional conditions such as those described in Sambrook et al., Molecular Cloning: A Laboratory Manual (New York: Cold Spring Harbor Laboratory Press, 1989) or conditions recommended by the manufacturer of a device are followed.

### Definitions and Description:

As used herein, the terms "comprise", "have", "include", or "contain" are intended to be inclusive or open-ended and do not exclude additional, unreferenced elements or method steps.

As used herein, "about" means that a value includes the standard deviation of error for the device or method used to determine the value.

As used herein, the definition of "or" is merely an alternative and "and/or", but unless it is explicitly to be only an alternative or mutually exclusive between alternatives, the term "or" in the claims refers to "and/or".

As used herein, the selected/optional/preferred "numerical range" includes both numerical endpoints at both ends of the range and all natural numbers covered between the numerical endpoints with respect to the aforementioned numerical endpoints.

In the present disclosure, the term "polynucleotide" refers to a polymer composed of nucleotides. Polynucleotide may be in the form of an individual fragment, or may be a component of a larger nucleotide sequence structure, which is derived from a nucleotide sequence isolated at least once in quantity or concentration and can be recognized, manipulated, and restored to the sequence and its component nucleotide sequences by standard molecular biology methods (e.g., using cloning vectors). When a nucleotide sequence is represented by a DNA sequence (i.e., A, T, G, C), this also includes an RNA sequence (i.e., A, U, G, C) in which "U" substitutes for "T". In other words, a "polynucleotide" refers to a polymer of nucleotides removed from other nucleotides (either an individual fragment or an entire fragment), or may be a component or constituent of a larger nucleotide structure, such as an expression vector or a polycistronic sequence. Polynucleotides include DNA, RNA, and cDNA sequences.

In the present disclosure, the term "mutation" means that nucleotide(s) at one or more (e.g., several) positions of a polynucleotide are mutated, and the promoter activity of the polynucleotide is maintained. In the present disclosure, "mutation" (including substitution, insertion and/or deletion) particularly refers to a substitution and a deletion, and the substitution refers to the replacement of a nucleotide occupying one position with a different nucleotide. Deletion refers to the removal of a nucleotide occupying a certain position.

In some specific embodiments, the "mutation" of the present disclosure comprises a substitution and/or deletion of nucleotide(s) at one or more positions in a partial sequence TTACTCTA (i.e., positions 366 to 373 of the sequence set forth in SEQ ID NO: 1) in the RBS spacer of the sequence set forth in SEQ ID NO: 1. Compared with the unmodified SEQ ID NO: 1, a polynucleotide sequence formed by linking the mutated sequence to a start codon GTG or TTG has the activity of enhancing *lysC* expression.

Illustratively, the "mutation" of the present disclosure comprises a substitution and/or deletion at 1, 2, 3, 4, 5, 6, 7 or 8 positions at positions 366 to 373 of the sequence set forth in SEQ ID NO: 1. The "mutation" of the present disclosure comprises a deletion of 6 nucleotides at positions 363 to 373 of the sequence set forth in SEQ ID NO: 1, and/or a substitution of 0-2 nucleotides of the deleted sequence, and a linkage to a start codon GTG or TTG. SEQ ID NO: 2, SEQ ID NO: 3, or SEQ ID NO: 4 formed after the above mutations has higher transcriptional expression regulatory activity as compared with the unsubstituted SEQ ID NO: 1.

The term "promoter" in the present disclosure refers to a nucleic acid molecule, which is generally located upstream of a coding sequence of a target gene, providing a recognition site for RNA polymerase, and is located upstream in the 5' direction of the mRNA transcription start site. It is an untranslated nucleic acid sequence to which RNA polymerase binds to initiate the transcription of the target gene. In the synthesis of ribonucleic acid (RNA), the promoter may interact with a transcription factor that regulates gene transcription, controlling the initiation time and extent of gene expression (transcription), comprising a core promoter region and a regulatory region. The promoter functions like a "switch" that determines the activity of a gene and thus controls which protein the cell starts to produce.

The term "RBS spacer" in the present disclosure refers to a nucleic acid sequence located in the promoter region of a prokaryote, which is a nucleotide sequence between the RBS and the start codon.

The term "start codon" in the present disclosure has a definition well known to those skilled in the art, which refers to a codon at the start site of protein synthesis, and generally includes ATG, GTG, TTG and ATA. Illustratively, in the present disclosure, the start codon contained in SEQ ID NO: 2 and SEQ ID NO: 3 is GTG, and the start codon contained in SEQ ID NO: 4 is TTG.

The term "expression" in the present disclosure includes any steps involving RNA production and protein production, including, but not limited to: transcription, post-transcriptional modification, translation, post-translational modification, and secretion.

The term "transcriptional expression cassette" in the present disclosure refers to a type of expression element comprising a transcription regulatory element and a target gene, wherein the transcription regulatory element is used to regulate the expression of the target gene. In the present disclosure, the transcription regulatory element comprises a promoter, and on this basis, it may also comprise an enhancer, a silencer, an insulator, and the like. In the present disclosure, the target gene is specifically a protein-encoding gene.

The "operable linkage" of the target gene to the polynucleotide refers to functionally linking the polynucleotide having promoter activity to the target gene so as to initiate and mediate the transcription of the target gene. The operable linkage can be performed by any method described by those skilled in the art. Illustratively, the operable linkage of the target gene to the polynucleotide sequence of the present disclosure is a polynucleotide sequence formed by operably linking SEQ ID NO: 2, SEQ ID NO: 3, or SEQ ID NO: 4 to gene *lysC.*

The "gene *lysC*" in the present disclosure refers to a coding gene of aspartate kinase (EC: 2.7.2.4) derived from *C. glutamicum,* including, but not limited to, naturally occurring *lysC* gene derived from *C. glutamicum, lysC* gene mutants (e.g., T311I, D274A, Q298G, N299L, N374A, E382A, R384L, etc.) that have been relieved of lysine feedback inhibition, and *lysC* gene mutants derived from *C. glutamicum* that have been artificially modified but still have aspartate kinase activity. Illustratively, the gene *lysC* of the present disclosure is an aspartate kinase-encoding gene of *C. glutamicum* ATCC13032, which has a gene number of NCgl0247 or Cgl0251 and an NCBI database Gene ID of 1021294, and encodes a protein having a Protein ID of NP_599504 or WP_003855724.1.

The "gene *lysC* without a start codon" of the present disclosure refers to a coding gene of aspartate kinase (EC: 2.7.2.4) in which the start codon GTG is deleted.

The term "vector" in the present disclosure refers to a DNA construct, containing a DNA sequence operably linked to a suitable control sequence for the expression of a target gene in a suitable host. "Recombinant expression vector" refers to a DNA construct used to express, for example, a polynucleotide encoding a desired polypeptide. Recombinant expression vectors may include, for example, vectors comprising i) a collection of genetic elements that have a regulatory effect on gene expression, such as promoters and enhancers; ii) a structure or coding sequence that is transcribed into mRNA and translated into a protein; and iii) transcriptional subunits of appropriate transcriptional and translational initiation and termination sequences. The recombinant expression vector is constructed in any suitable manner. The nature of the vector is not critical and any vector may be used, including plasmids, viruses, phages and transposons. Potential vectors for use in the present disclosure include, but are not limited to, chromosomal, non-chromosomal and synthetic DNA sequences, such as bacterial plasmids, phage DNAs, yeast plasmids, and vectors derived from combinations of plasmids and phage DNAs, and DNAs from viruses such as vaccinia, adenovirus, fowl pox, baculovirus, SV40 and pseudorabies.

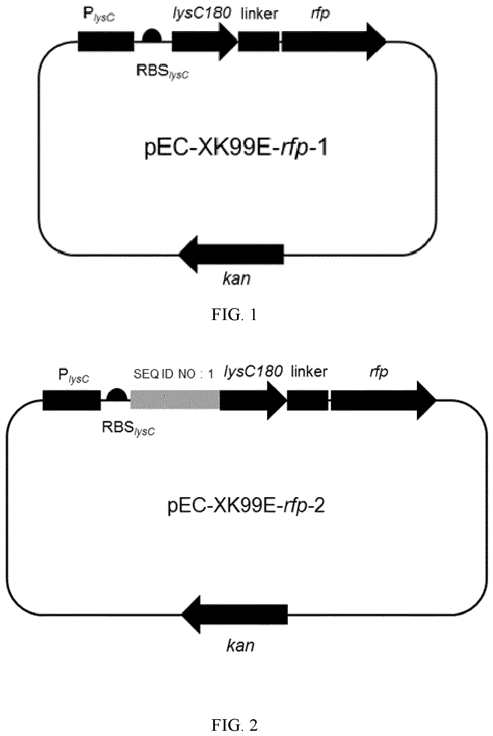

Illustratively, vectors involved in the present disclosure are characterization plasmids pEC-XK99E-*rfp*-1 and pEC-XK99E-*rfp*-2 having expression regulatory sequences constructed based on pEC-XK99E-*rfp* plasmid. Plasmid maps of pEC-XK99E-*rfp*-1 and pEC-XK99E-*rfp*-2 are shown in FIG. 1 and FIG. 2, respectively. In FIG. 1, P_{lysC} indicates a wild-type promoter of *lysC* gene; lysC180 indicates a 180-bp coding region at the N-terminal of the wild-type aspartate kinase LysC-encoding gene; linker indicates a linker peptide between *lysC* gene and *rfp* protein; *rfp* indicates red fluorescent protein (RFP); and Kan indicates Kanamycin resistance. In FIG. 2, SEQ ID NO: 1 indicates a promoter set forth in SEQ ID NO: 1; lysC180 indicates a 180-bp coding region at the N-terminal of the wild-type aspartate kinase LysC-encoding gene; linker indicates a linker peptide between *lysC* gene and *rfp* protein; *rfp* indicates red fluorescent protein (RFP); and Kan indicates Kanamycin resistance. After being transformed into a suitable host, pEC-XK99E-*rfp*-1 and pEC-XK99E-*rfp*-2 can replicate and function independently of a genome of the host or in some cases integrate into the genome itself.

The term "host cell" in the present disclosure means any type of cell which is susceptible to transformation, transfection, transduction, and the like with a transcription initiation element or expression vector comprising a polynucleotide of the present disclosure. The term "recombinant host cell" encompasses a host cell which differs from the parent cell after introduction of a transcription initiation element or a recombinant expression vector, and specifically, the recombinant host cell can be achieved by transformation.

The term "transformation" in the present disclosure has the meaning generally understood by those skilled in the art, i.e., the process of introducing exogenous DNA into a host. Methods for the transformation include any method for introducing a nucleic acid into a cell, including, but not limited to, electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method.

The host cell of the present disclosure is a prokaryotic cell, as long as it is a cell into which the polynucleotides having transcriptional expression regulatory activity of the present disclosure can be introduced. In one embodiment, the host cell is derived from a microorganism suitable for fermentative production of amino acids, such as *Corynebacterium, Brevibacterium, Arthrobacter, Microbacterium,* or *Escherichia.* Preferably, the host cell is *C. glutamicum* derived from the genus *Corynebacterium,* wherein the *C. glutamicum* may be *C. glutamicum* ATCC 13032, *C. glutamicum* ATCC 13869, or *C. glutamicum* ATCC 14067 etc., or a derivative strain thereof. Illustratively, the derivative strain may be any strain as long as the strain has the ability to produce an L-amino acid.

Illustratively, the host cell is a lysine-producing host cell. In some embodiments, as for the lysine-producing host cell, it may be a derivative strain on the basis of *C. glutamicum* ATCC 13032, which expresses aspartate kinase relieved of feedback inhibition. In addition, the lysine-producing host cell may also be a strain of another species having lysine-producing ability.

In some embodiments, the lysine-producing host cell may also comprise, but is not limited to, one or more genes selected from the following being attenuated or decreased in expression:
a. adhE gene encoding alcohol dehydrogenase;
b. ackAgene encoding acetate kinase;
c. pta gene encoding phosphate acetyltransferase;
d. ldhA gene encoding lactate dehydrogenase;
e. focA gene encoding formate transporter;
f. pflB gene encoding pyruvate formate lyase;
g. poxB gene encoding pyruvate oxidase;
h. thrA gene encoding aspartate kinase I/homoserine dehydrogenase I bifunctional enzyme;
i. thrB gene encoding homoserine kinase;
j. ldcC gene encoding lysine decarboxylase; and
h. cadA gene encoding lysine decarboxylase.

In some embodiments, the lysine-producing host cell may also comprise, but is not limited to, one or more genes selected from the following being enhanced or overexpressed:
a. dapA gene encoding dihydrobipyridine synthase that is relieved of feedback inhibition by lysine;
b.dapB gene encoding dihydrodipicolinate reductase;
c. ddh gene encoding diaminopimelate dehydrogenase;
d. dapD encoding tetrahydrodipicolinate succinylase and dapE encoding succinyldiaminopimelate deacylase;
e. asd gene encoding aspartate-semialdehyde dehydrogenase;
f. ppc gene encoding phosphoenolpyruvate carboxylase;
g. pntAB gene encoding nicotinamide adenine dinucleotide transhydrogenase; and
i. lysE gene encoding transport protein of lysine.

Illustratively, the host cell is a threonine-producing host cell. In some embodiments, the threonine-producing host cell is a strain on the basis of *C. glutamicum* ATCC 13032, which expresses aspartate kinase LysC relieved of feedback inhibition. In other embodiments, the threonine-producing host cell may also be a strain of another species having threonine-producing ability.

In some embodiments, in the threonine-producing host cell, one or more genes selected from the following are enhanced or overexpressed:
a. thrABC gene encoding threonine operon;
b. horn gene encoding homoserine dehydrogenase that is relieved of feedback inhibition;
c. gap gene encoding glyceraldehyde-3-phosphate dehydrogenase;
d. pyc gene encoding pyruvate carboxylase;
e. mqo gene encoding malate:quinone oxidoreductase;
f. tkt gene encoding transketolase;
g. gnd gene encoding 6-phosphogluconate dehydrogenase;
h. thrE gene encoding threonine exporter; and
i. eno gene encoding enolase.

Illustratively, the host cell is an isoleucine-producing host cell. In some embodiments, the isoleucine-producing host cell is a strain that produces L-isoleucine by substituting the amino acid at position 323 of the ilvA gene of L-threonine dehydratase with alanine. In other embodiments, the isoleucine-producing host cell may also be a strain of another species having isoleucine-producing ability.

Illustratively, the host cell is an O-acetylhomoserine-producing host cell. In some embodiments, the O-acetylhomoserine-producing host cell is a strain that produces O-acetylhomoserine by inactivating O-acetylhomoserine(thiol)-lyase. In other embodiments, the O-acetylhomoserine-producing host cell may also be a strain of another species having O-acetylhomoserine-producing ability.

Illustratively, the host cell is a methionine-producing host cell. In some embodiments, the methionine-producing host cell is a strain that produces methionine by inactivating the transcriptional regulators of methionine and cysteine. In other embodiments, the methionine-producing host cell may also be a strain of another species having methionine-producing ability.

The culture of the host cell of the present disclosure may be performed according to a conventional method in the art, including, but not limited to, well-plate culture, shake-flask culture, batch culture, continuous culture, fed-batch culture, and the like, and various culture conditions such as temperature, time, pH of a medium, and the like may be appropriately adjusted according to actual circumstances.

Unless defined otherwise, or clear from the background, all technical and scientific terms used in the present disclosure have the same meaning as commonly understood by one of ordinary skill in the art to which the present disclosure belongs.

### Polynucleotides having activity of enhancing expression of gene lysC

In the present disclosure, characterization vectors pEC-XK99E-*rfp*-3, pEC-XK99E-*rfp*-4, and pEC-XK99E-*rfp*-5 are obtained by modifying TTACTCTA**GTG** (the underlined sequence is a sequence at positions 366 to 373 of SEQ ID NO:1, which is a partial sequence in the RBS spacer; the bold sequence is the start codon of gene *lysC*) in pEC-XK99E-rfp-2 plasmid into sequences "AG**GTG**", "TC**GTG**", and "TT**TTG**", respectively, and cloning and ligating the sequences containing the mutations described above with pEC-XK99E plasmid backbone fragments through a one-step cloning kit from Vazyme. The plasmids described above are transformed into *C. glutamicum* ATCC13032 to obtain ATCC13032 (pEC-XK99E-*rfp*-3), ATCC13032 (pEC-XK99E-*rfp*-4), and ATCC13032 (pEC-XK99E-*rfp*-5) strains, respectively. The above three strains have higher regulatory activity on gene *lysC* transcriptional expression as compared with the control strain ATCC13032 (pEC-XK99E-*rfp*-2).

### Process for producing lysine

(1) In the present disclosure, polynucleotides having the activity of enhancing the expression of gene *lysC* are each operably linked to a coding gene of an enzyme involved in the synthesis of amino acid, and recombinant expression vectors capable of synthesizing the enzyme involved in the synthesis of the amino acid are obtained and then transformed into host cells to obtain recombinant host cells.
(2) Fermentation culture is performed on the recombinant host cells, and lysine is collected from the recombinant host cells or a culture solution of the recombinant host cells, such that the process for producing lysine is completed.

In some embodiments of the present disclosure, the host cell is *C. glutamicum.* In some specific embodiments of the present disclosure, the recombinant host cell is *C. glutamicum* modified as follows: 1) a mutation coding sequence of T311I is introduced into an aspartate kinase-encoding gene *lysC* in *C. glutamicum* ATCC13032; 2) a core region from position 279 to position 317 of the pyruvate carboxylase gene *pyc* promoter in *C. glutamicum* is CGGGCCTTGATTGTAAGATAAGACATTTAGTATAATTAG; 3) nucleotides from position 279 to position 317 of the diaminopimelate dehydrogenase gene *ddh* promoter in *C. glutamicum* are mutated from wild type ATGCATCTC to CCTTGTTAT; and 4) recombinant vector pK18-1, pK18-2, pK18-3, or pK18-4 containing polynucleotide SEQ ID NO: 2, SEQ ID NO: 3, or SEQ ID NO: 4 with the transcriptional expression regulatory activity is transformed into *C. glutamicum* described above to obtain recombinant *C. glutamicum.* The *C. glutamicum* modified by the method described above is a strain with high yield of L-lysine. The host cell according to the present disclosure has an improvement in the expression of the aspartate kinase gene compared to the wild type. Since aspartate kinase is the most important enzyme in the biosynthetic pathway of lysine, fermentation of the host cell results in a higher yield of lysine. In the present disclosure, fermentation of the transformant can be carried out by a well-known method, and conditions for the fermentation including temperature, time, pH, and the like can be appropriately controlled. The following documents provide a detailed description of fermentation [Chmiel; Bioprozesstechnik 1. Einffuhrung in die Bioverfahrenstechnik (Gustav Fischer Verlag, Stuttgart, 1991), and Storhas; Bioreaktoren und periphere Einrichtungen (Vieweg Verlag, Braunschweig/Wiesbaden, 1994)]. Fermentation can be achieved by batch culture, continuous culture, or fed-batch culture.

For fermentation, the medium must meet the requirements of the strains used. The medium suitable for culturing various microorganisms is well known in the art (e.g., "Manual of Methods for General Bacteriology" from American Society for Bacteriology (Washington D.C., USA, 1981)). The medium may contain, for example, saccharides and carbohydrates (e.g. glucose, sucrose, lactose, fructose, maltose, molasses, starch, and cellulose), lipids and fats (e.g. soya oil, sunflower oil, peanut oil, and coconut oil), fatty acids (e.g. palmitic acid, stearic acid, and ricinoleic acid), alcohols (e.g. glycerol and ethanol), and organic acids (e.g. acetic acid). These substances may be used separately or in combination. As nitrogen sources, nitrogen-containing organic compounds (e.g., peptone, yeast extract, broth, wort, corn steep liquor, soybean meal, and urea) or nitrogen-containing inorganic compounds (e.g., ammonium sulfate, ammonium chloride, ammonium phosphate, ammonium carbonate, and ammonium nitrate) may be used separately or in combination. Examples of phosphorus sources for the medium include dipotassium hydrogen phosphate, potassium dihydrogen phosphate, and the corresponding sodium salts.

In addition, the medium may contain metal salts (e.g., magnesium sulfate or iron sulfate) necessary for cell growth, and may be supplemented with essential nutrients such as amino acids and vitamins to stimulate cell growth. In addition, appropriate precursors may be added to the medium. Nutrients and additives may be added together at once or separately during the fermentation process.

The pH of the medium can be adjusted with an alkaline compound (e.g., sodium hydroxide, potassium hydroxide or ammonia) or an acidic compound (e.g., phosphoric acid or sulfuric acid). The formation of foam in the medium can be suppressed by using an antifoaming agent such as polyethylene glycol fatty acid ester. The medium can be maintained under aerobic conditions by introducing oxygen or an oxygen-containing gas mixture therein. As for the culture temperature, it is usually between 20 °C and 45 °C, preferably between 25 °C and 40 °C. The fermentation proceeds continuously until a maximum amount of L-lysine is produced. In this regard, it can be completed in 10 to 160 hours. After L-lysine is produced, L-lysine can be exported to the medium or can be kept inside the cell.

In some specific embodiments of the present disclosure, the recombinant host cells are cultured under the following conditions: the strain is first inoculated into TSB liquid medium and cultured for 8 h, and then the culture is inoculated as a seed into a 24-well plate containing 800 µl of a fermentation medium per well, with an initial OD₆₀₀ controlled at approximately 0.1, and cultured at 30 °C for 19 h with the rotation speed of a well-plate shaker being 800 rpm.

The TSB liquid medium contains the following components: glucose, 5 g/L; yeast powder, 5 g/L; soybean peptone, 9 g/L; urea, 3 g/L; succinic acid, 0.5 g/L; K₂HPO₄•3H₂O, 1 g/L; MgSO₄•7H₂O, 0.1 g/L; biotin, 0.01 mg/L; vitamin B1, 0.1 mg/L; and MOPS, 20 g/L.

The fermentation medium contains the following components: glucose, 80 g/L; yeast powder, 1 g/L; soybean peptone, 1 g/L; NaCl, 1 g/L; ammonium sulfate, 1 g/L; urea, 8 g/L; K₂HPO₄•3H₂O, 1 g/L; MgSO₄•7H₂O, 0.45 g/L; FeSO₄•7H₂O, 0.05 g/L; biotin, 0.4 mg/L; vitamin B1, 0.1 mg/L; and MOPS, 40 g/L, with the initial pH being 7.2.

In some specific embodiments, the amino acid is recovered from the recombinant host cells or the culture solution of the recombinant cells by methods commonly used in the art, including, but not limited to: filtration, anion exchange chromatography, crystallization, and HPLC.

### Example 1. Enhancement of Expression of Gene lysC in C. Glutamicum by Artificial Expression Regulatory Sequence

In bacteria, the expression regulatory sequence and the N-terminal coding region of the gene are the key regions affecting gene expression. In the present disclosure, the expression intensity of the expression regulatory sequences of gene *lysC* was characterized on the basis of the fluorescence intensity by adopting a sequential linkage of an expression regulatory region of the gene, a 180-bp coding region at the N-terminal of the gene, a flexible linker and a red fluorescent protein gene *rfp.*

In this example, one vector was constructed first for characterizing the expression intensity of the self-expression regulatory sequence of gene *lysC* in C. *glutamicum.* The construction was carried out as follows: On the basis of pEC-XK99E plasmid backbone, 60 amino acids at the N-terminal of gene *lysC,* a linker peptide and a red fluorescent protein gene were expressed by gene *lysC* self-promoter and RBS. Based on the published genome sequence (Gene ID: 2830649) of *C. glutamicum* ATCC13032 and the annotation information of gene *lysC,* a primer lysC-F/R was designed, and a DNA fragment containing the gene *lysC* promoter, RBS, and N-terminal 180-bp fragment was obtained by PCR amplification using ATCC13032 genome as a template. A DNA fragment containing the pEC-XK99E plasmid backbone, linker peptide fragment (DNA sequence: GGCGGTGGCTCTGGAGGTGGTGGGTCCGGCGGTGGCTCT) and red fluorescent protein gene fragment was amplified using pEC-XK99E-*rfp* plasmid reported in the literature (Wang Y C, et al. Screening efficient constitutive promoters in Corynebacterium glutamicum based on time-series transcriptome analysis[J]. Chinese Journal of Biotechnology, 2018, 34(11): 1760-1771) as a template and pEC-1/2 as a primer. The two fragments described above were cloned and ligated by a one-step cloning kit from Vazyme to obtain a pEC-XK99E-*rfp*-1 characterization vector with a plasmid map shown in FIG. 1.

To enhance the expression intensity of the gene *lysC,* an expression regulatory sequence set forth in SEQ ID NO: 1 was synthesized artificially in this example and was inserted upstream of the gene *lysC.* To characterize the effect of inserting the artificial expression regulatory sequence to enhance the expression intensity, a characterization vector was further constructed on the basis of pEC-XK99E-*rfp*-1 characterization vector. The construction was carried out as follows: The expression regulatory sequence with the nucleotide sequence set forth in SEQ ID NO: 1 synthesized by the DNA synthesis company was used as a template to amplify an artificial sequence with a 20-bp homology arm using *pyc*-FIR as a primer. The pEC-XK99E-*rfp*-1 plasmid was used as a template to amplify a plasmid backbone using pEC-3/4 as a primer. The two fragments described above were cloned and ligated by a one-step cloning kit from Vazyme to obtain a pEC-XK99E-*rfp*-2 characterization vector with a plasmid map shown in FIG. 2. The sequences of the primers used in this example are shown in Table 1.

**Table 1**

| Primer | Nucleotide sequence | SEQ ID NO. |
|---|---|---|
| *lysC-*F | CCTGATGCGGTATTTTCTCCGATTCAGGGTAGTTG ACTAAAGAGTTG | SEQ ID NO: 5 |
| *lysC-*R | AACGGGATTCACTGCCGCTG | SEQ ID NO: 6 |
| pEC-1 | CAGCGGCAGTGAATCCCGTTGGCGGTGGCTCTGG AGGTGGTGGGTCCGGCGGTGGCTCTGCTTCCTCC GAAGACGTTATCAAAG | SEQ ID NO: 7 |
| pEC-2 | GGAGAAAATACCGCATCAGGC | SEQ ID NO: 8 |
| *pyc-*F | GATCGAAAGGTGCACAAAGGGAAAACCCAGGAT TGCTTTGTG | SEQ ID NO: 9 |
| *pyc-*R | TTCTGTACGACCAGGGCCACTAGAGTAATTATTCC TTTCAACAAGAG | SEQ ID NO: 10 |
| pEC-3 | GTGGCCCTGGTCGTACAGAAATATG | SEQ ID NO: 11 |
| pEC-4 | CCTTTGTGCACCTTTCGATCTAG | SEQ ID NO: 12 |

To characterize the intensity of the expression enhanced by insertion of the artificial expression regulatory sequence (SEQ ID NO: 1), the constructed pEC-XK99E-*rfp*-1 and pEC-XK99E-*rfp*-2 were separately transformed into C. *glutamicum* ATCC13032 to obtain ATCC13032 (pEC-XK99E-*rfp*-1) and ATCC13032 (pEC-XK99E-*rfp*-2) strains. 96-well plate culture was used to characterize the intensity of the artificial expression regulatory sequence (SEQ ID NO: 1) expressing *lysC.* The TSB liquid medium contained the following components (g/L): glucose, 5 g/L; yeast powder, 5 g/L; soybean peptone, 9 g/L; urea, 3 g/L; succinic acid, 0.5 g/L; K₂HPO₄·3H₂O, 1 g/L; MgSO₄·7H₂O, 0.1 g/L; biotin, 0.01 mg/L; vitamin B1, 0.1 mg/L; and MOPS, 20 g/L. The TSB solid medium was supplemented with an additional 15 g/L agar powder. The strains obtained from the TSB plates were inoculated with toothpicks into 96-well plates containing 200 µL of TSB liquid medium per well, and the experiment for each strain was performed in triplicate. The rotation speed of the well-plate shaker was 800 rpm, and the fluorescence intensity of the strains was measured with a microplate reader (excitation wavelength: 560 nm, emission wavelength: 607 nm) after culturing at 30 °C for 24 h. As can be seen from the measurement results shown in Table 2, the insertion of the artificial expression regulatory sequence (SEQ ID NO: 1) increased the fluorescence intensity by 4.8-fold, indicating that the insertion of the artificial expression regulatory sequence described above could enhance the expression intensity of gene *lysC.*

**Table 2**

| Strain | Fluorescence intensity (RFU/OD₆₀₀) |
|---|---|
| ATCC13032 (pEC-XK99E-*rfp*-1) | 277±3 |
| ATCC13032 (pEC-XK99E-*rfp*-2) | 1613±38 |

### Example 2. Further Enhancement of Expression Intensity of Gene lysC by Modification of Artificial Expression Regulatory Sequences and Gene lysC Start Codon

The modification of the base sequence of RBS spacer and the start codon can be used to further enhance the expression intensity. In this example, the TTACTCTA**GTG** (the underlined sequence is a sequence at positions 366 to 373 of SEQ ID NO:1, which is a partial sequence in the RBS spacer; the bold sequence is the start codon of gene *lysC*) in the pEC-XK99E-*rfp*-2 plasmid was modified to sequences "AG**GTG**", "TC**GTG**" and "TT**TTG**", and the polynucleotide sequences with expression regulatory activity obtained after the modification are set forth in SEQ ID NO: 2, SEQ ID NO: 3, and SEQ ID NO: 4, respectively. The construction was carried out as follows: The pEC-XK99E-*rfp*-2 plasmid was used as a template, and RBS1/ pEC-5, RBS2/ pEC-5, and RBS3/ pEC-5 were used as primers to amplify three fragments including three modified regions, respectively. The pEC-XK99E-*rfp*-2 plasmid was used as a template to amplify a plasmid backbone using pEC-6/7 as a primer. The three fragments including the modified regions described above were separately cloned and ligated with the plasmid backbone fragment through a one-step cloning kit from Vazyme to obtain pEC-XK99E-*rfp*-3, pEC-XK99E-*rfp*-4, and pEC-XK99E-*rfp*-5 characterization vectors, respectively. The sequences of the primers used in this example are shown in Table 3.

**Table 3**

| Primer | Nucleotide sequence | SEQ ID NO. |
|---|---|---|
| RBS-1 | CGGTCTCTTGTTGAAAGGAATAAAGGTGGCCCTG GTCGTACAGAAATATG | SEQ ID NO: 13 |
| RBS-2 | CGGTCTCTTGTTGAAAGGAATAATCGTGGCCCTG GTCGTACAGAAATATG | SEQ ID NO: 14 |
| RBS-3 | CGGTCTCTTGTTGAAAGGAATAATTTTGGCCCTG GTCGTACAGAAATATG | SEQ ID NO: 15 |
| pEC-5 | AACCTTCCATACGAACTTTGAAACG | SEQ ID NO: 16 |
| pEC-6 | CAAAGTTCGTATGGAAGGTTCCG | SEQ ID NO: 17 |
| pEC-7 | TTCCTTTCAACAAGAGACCGCC | SEQ ID NO: 18 |

To characterize the intensity of the expression enhanced by SEQ ID NO: 2, SEQ ID NO: 3, and SEQ ID NO: 4, pEC-XK99E-*rfp*-3, pEC-XK99E-*rfp*-4 and pEC-XK99E-*rfp*-5 plasmids were separately transformed into C. *glutamicum* ATCC13032 to obtain ATCC13032 (pEC-XK99E-*rfp*-3), ATCC13032 (pEC-XK99E-*rfp*-4) and ATCC13032 (pEC-XK99E-*rfp*-5) strains. The fluorescence intensity of the strains described above and the control strain ATCC13032 (pEC-XK99E-*rfp*-2) was measured by the same strategy as in Example 1. As can be seen from the results shown in Table 4, the fluorescence intensity of ATCC13032 (pEC-XK99E-*rfp*-3), ATCC13032 (pEC-XK99E-*rfp*-4) and ATCC13032 (pEC-XK99E-*rfp*-5) strains increased 1.3-fold, 2.7-fold, and 5.0-fold, respectively, compared with the control strain, indicating that the expression of gene *lysC* could be further enhanced by the modification of the start codon and upstream RBS spacer sequence.

**Table 4**

| Strain | Fluorescence intensity (RFU/OD₆₀₀) |
|---|---|
| ATCC13032 (pEC-XK99E-*rfp*-2) | 1711±66 |
| ATCC13032 (pEC-XK99E-*rfp*-3) | 3928±963 |
| ATCC13032 (pEC-XK99E-*rfp*-4) | 6280±1324 |
| ATCC13032 (pEC-XK99E-*rfp*-5) | 8509±144 |

### Example 3. Application of Modified Expression Regulatory Sequences to L-Lysine Production

### (1) Construction of recombinant vectors modified with gene lysC expression regulatory sequences in C. glutamicum

According to the expression intensity characterized in Examples 1 and 2, SEQ ID NO: 2, SEQ ID NO: 3, and SEQ ID NO: 4 were separately inserted upstream of the gene *lysC* on the genome of *C. glutamicum* ATCC13032 in the same manner as before to enhance the expression of the gene *lysC.* According to the reported genome sequence of *C. glutamicum* ATCC13032, a portion of upstream homology arm was amplified by PCR using ATCC13032 genome as a template and *lysC-UF*/*lysC*-UR as a primer; corresponding expression regulatory sequences and portions of upstream and downstream homologous arms were separately amplified by using pEC-XK99E-*rfp*-3, pEC-XK99E-*rfp*-4, and pEC-XK99E-*rfp*-5 plasmids as templates and *Pyc*-RBS1/*Pyc*-RBS2 as a primer, respectively; another portion of downstream homology arm was amplified by PCR using *lysC-DF*/*lysC-DR* as a primer; and the backbone of pK18mobsacB (GenBank: FJ437239.1) was amplified by using pK18-1F/2R as a primer. After the 4 PCR fragments described above were recovered, recombinant vectors pK18-2, pK18-3, and pK18-4 modified with the expression regulatory sequences of gene *lysC* were obtained by cloning and ligating through a one-step cloning kit from Vazyme, respectively. The sequences of the primers used above are shown in Table 5.

**Table 5**

| Primer | Nucleotide sequence | SEQ ID NO. |
|---|---|---|
| *lysC-*UF | CAGGAAACAGCTATGACATGCGTCACAA GACCAAGGATG | SEQ ID NO: 19 |
| *lysC-*UR | AAGTGCAAAGCAATCTGCTC | SEQ ID NO: 20 |
| *Pyc*-RBS1 | GAGCAGATTGCTTTGCACTTGATTCAGG GTAGTTGACTAAAGAG | SEQ ID NO: 21 |
| *Pyc*-RBS2 | AACGGGATTCACTGCCGC | SEQ ID NO: 22 |
| *lysC*-DF | GCGGCAGTGAATCCCGTTCCGCCAGCTC GTGAAAT | SEQ ID NO: 23 |
| *lysC*-DR | GTAAAACGACGGCCAGTGCGGAGCCAA | SEQ ID NO: 24 |
| | CAGCAGCAAG | |
| pK18-1F | GCACTGGCCGTCGTTTTAC | SEQ ID NO: 25 |
| pK18-2R | CATGTCATAGCTGTTTCCTGTGTG | SEQ ID NO: 26 |

### (2) Introduction of recombinant vectors modified with gene lysC expression regulatory sequences into C. glutamicum lysine-producing bacteria

To verify the effect of gene *lysC* expression sequence modification on L-lysine production, in the present disclosure, a basic strain SCgL40 that can produce L-lysine was first constructed. The genetic modification of *C. glutamicum* L-lysine-producing bacterium SCgL40 included the introduction of a T311I (bases mutated from ACC to ATC) amino acid mutation in *C. glutamicum* ATCC13032 aspartate kinase gene *lysC,* the mutation of nucleotides from position 279 to position 317 of the pyruvate carboxylase gene pyc promoter from wild type CGATGTTTGATTGGGGGAATCGGGGGTTACGATACTAGG to CGGGCCTTGATTGTAAGATAAGACATTTAGTATAATTAG, and the mutation of nucleotides from position 279 to position 317 of the diaminopimelate dehydrogenase gene *ddh* promoter from wild type ATGCATCTC to CCTTGTTAT. To further introduce gene *lysC* expression regulatory sequence modification, the recombinant vectors pK18-2, pK18-3, and pK18-4 constructed in (1) above were separately transformed into *C. glutamicum* lysine-producing strain SCgL40, and then the bacteria were applied on LBHIS solid medium containing 5 g/L glucose and 25 µg/mL kanamycin, and cultured at 30 °C to obtain primary recombinant transformants. The correct primary recombinant transformants were inoculated separately to LB medium containing 5 g/L glucose and cultured overnight. The cultures were diluted and applied separately to LB solid medium plates supplemented with 100 g/L sucrose, and the clones obtained were screened for kanamycin sensitivity by simultaneous spot-plate culture on resistant and non-resistant plates. The kanamycin-sensitive clones were PCR amplified and sequenced using primers lysC-C1 (sequence: CCCAGTTCAAGATGAGTCCC) and lysC-C2 (sequence: CCGGGATCATTACTATAAGACG), respectively. The correctly sequenced clones were the obtained strains SCgL42, SCgL43, and SCgL44 with modified gene *lysC* expression sequences.

### (3) Evaluation of L-lysine-producing ability of mutants of C. glutamicum lysine-producing bacterium with modified gene lysC expression elements

To test the effect of gene *lysC* expression element modification in *C. glutamicum* on the production of L-lysine by the strains, fermentation tests were performed on SCgL40, SCgL42, SCgL43, and SCgL44 strains, respectively. The fermentation medium contained the following components: glucose, 80 g/L; yeast powder, 1 g/L; soybean peptone, 1 g/L; NaCl, 1 g/L; ammonium sulfate, 1 g/L; urea, 8 g/L; k₂HPO₄·3H₂O, 1 g/L; MgSO₄·7H₂O, 0.45 g/L; FeSO₄·7H₂O, 0.05 g/L; biotin, 0.4 mg/L; vitamin B1, 0.1 mg/L; and MOPS, 40 g/L, with the initial pH being 7.2. The strains were first inoculated into TSB liquid medium and cultured for 8 h, and then the cultures were inoculated as seeds into 24-well plates containing 800 µL of fermentation medium per well, with an initial OD₆₀₀ controlled at approximately 0.1, and cultured at 30°C for 19 h with the rotation speed of a well-plate shaker being 800 rpm. The experiment for each strain was performed in triplicate. The L-lysine yield and glucose consumption were detected at the end of fermentation, and the conversion rate from glucose to L-lysine was calculated. As can be seen from the results shown in Table 6, the lysine yield and the conversion rate from glucose to L-lysine of the strains with modified gene *lysC* expression elements were increased, wherein the lysine yield from the SCgL42 strain was increased to 89%. The results described above indicate that the expression sequences that enhanced the gene *lysC* expression intensity can be applied to L-lysine production.

**Table 6**

| Strain | OD₆₀₀ | L-lysine yield (g/L) | Conversion rate (%) |
|---|---|---|---|
| SCgL40 | 15.7±0.4 | 3.7±0.3 | 6.9±0.5 |
| SCgL42 | 15.9±0.7 | 7.0±1.0 | 14.0±2.0 |
| SCgL43 | 17.3±0.3 | 5.0±0.0 | 8.9±0.2 |
| SCgL44 | 10.9±0.3 | 4.7±0.6 | 14.1±1.0 |

Since aspartic acid downstream products are all synthesized in dependence on the reaction step catalyzed by aspartate kinase LysC, the method of gene *lysC* expression enhancement disclosed by the present disclosure can also be used to increase the yield of downstream products that are dependent on the LysC-catalyzed reaction. Therefore, the technical solutions provided by the present disclosure can also be used for producing L-threonine, L-isoleucine, L-homoserine, L-methionine, and downstream products of L-lysine, such as pentanediamine, 5-aminopentanoic acid, glutaric acid, and the like.

The embodiments of the present disclosure have been described above. However, the present disclosure is not limited to the embodiments described above. Any modification, equivalent, improvement, and the like made without departing from the spirit and principle of the present disclosure shall fall within the protection scope of the present disclosure.

## Claims

1. A polynucleotide for regulating the expression of aspartate kinase gene *lysC,* wherein a polynucleotide sequence of the polynucleotide is:
(1) a polynucleotide sequence formed by linking a sequence resulting from substitution and/or deletion of one or more nucleotides at positions 366 to 373 in a sequence set forth in SEQ ID NO: 1 to a start codon GTG;
or
(2) a polynucleotide sequence formed by linking a sequence resulting from substitution and/or deletion of one or more nucleotides at positions 366 to 373 in a sequence set forth in SEQ ID NO: 1 to a start codon TTG.

2. The polynucleotide sequence as claimed in claim 1, wherein the polynucleotide sequence has a nucleotide sequence set forth in SEQ ID NO: 2, SEQ ID NO: 3, or SEQ ID NO: 4.

3. A vector comprising the polynucleotide as claimed in claim 1 or 2.

4. The vector as claimed in claim 3, wherein the vector is based on pK18mobsacB as a backbone, and the GenBank accession number of the pK18mobsacB is FJ437239.1.

5. An expression cassette for regulating the expression of aspartate kinase gene *lysC,* wherein the expression cassette is a polynucleotide formed by operably linking the polynucleotide as claimed in claim 1 or 2 to an aspartate kinase gene *lysC* without a start codon.

6. A host cell comprising the polynucleotide as claimed in claim 1 or 2 or the expression cassette as claimed in claim 5.

7. The host cell as claimed in claim 6, wherein the host cell is derived from any one of *Corynebacterium, Brevibacterium, Arthrobacter, Microbacterium,* or *Escherichia.*

8. The host cell as claimed in claim 7, wherein the host cell is *C*. *glutamicum* or *Escherichia coli.*

9. The host cell as claimed in claim 8, wherein the host cell is *C*. *glutamicum* ATCC 13032, *C*. *glutamicum* ATCC 13869, or *C*. *glutamicum* ATCC 14067, or a derivative strain thereof.

10. The host cell as claimed in claim 9, wherein the host cell is *C*. *glutamicum* modified as follows: 1) a mutation coding sequence of T311I is introduced into an aspartate kinase-encoding gene *lysC* in *C*. *glutamicum* ATCC 13032; 2) a core region from position 279 to position 317 of a pyruvate carboxylase gene *pyc* promoter in C. *glutamicum* is mutated from wild type CGATGTTTGATTGGGGGAATCGGGGGTTACGATACTAGG to CGGGCCTTGATTGTAAGATAAGACATTTAGTATAATTAG; and 3) a nucleotide sequence from position 279 to position 317 of a diaminopimelate dehydrogenase gene *ddh* promoter in *C. glutamicum* is mutated from wild type ATGCATCTC to CCTTGTTAT.

11. A method for enhancing the expression of an aspartate kinase-encoding gene *lysC,* wherein the method is an operable linkage of the polynucleotide as claimed in claim 1 or 2 to a gene *lysC* without a start codon.

12. The method for enhancing the expression of the aspartate kinase-encoding gene *lysC* as claimed in claim 11, wherein the operable linkage is to introduce a vector containing the polynucleotide described above into a host cell, and to integrate the polynucleotide into a genome of the host cell by homologous recombination.

13. The method for enhancing the expression of the aspartate kinase-encoding gene *lysC* as claimed in claim 12, wherein the host cell is derived from *Corynebacterium, Brevibacterium, Arthrobacter, Microbacterium,* or *Escherichia.*

14. The method for enhancing the expression of the aspartate kinase-encoding gene *lysC* as claimed in claim 13, wherein the host cell is *C. glutamicum* or *Escherichia coli.*

15. The method for enhancing the expression of the aspartate kinase-encoding gene *lysC* as claimed in claim 14, wherein the host cell is *C. glutamicum* ATCC 13032, *C. glutamicum* ATCC 13869, or *C. glutamicum* ATCC 14067, or a derivative strain thereof.

16. The method for enhancing the expression of the aspartate kinase-encoding gene *lysC* as claimed in claim 15, wherein the host cell is *C. glutamicum* modified as follows: 1) a mutation coding sequence of T311I is introduced into the aspartate kinase-encoding gene *lysC* in *C. glutamicum* ATCC 13032; 2) a core region from position 279 to position 317 of a pyruvate carboxylase gene *pyc* promoter in *C. glutamicum* is mutated from wild type CGATGTTTGATTGGGGGAATCGGGGGTTACGATACTAGG to CGGGCCTTGATTGTAAGATAAGACATTTAGTATAATTAG; and 3) a nucleotide sequence from position 279 to position 317 of a diaminopimelate dehydrogenase gene *ddh* promoter in *C. glutamicum* is mutated from wild type ATGCATCTC to CCTTGTTAT.

17. Use of the polynucleotide sequence as claimed in claims 1-2, the vector as claimed in claim 3 or 4, the expression cassette as claimed in claim 5, and the host cell as claimed in any one of claims 6-10 in the production of L-lysine.

18. A method for producing L-lysine, wherein the method comprises culturing the host cell as claimed in any one of claims 6-10 to produce L-lysine and isolating L-lysine from a fermentation broth.

19. Use of the polynucleotide as claimed in claims 1-2, the vector as claimed in claim 3 or 4, the expression cassette as claimed in claim 5, and the host cell as claimed in any one of claims 6-10 in the preparation of aspartate-family amino acids and derivatives thereof.

20. The use as claimed in claim 19, wherein the aspartate-family amino acids and the derivatives thereof are L-threonine, L-isoleucine, L-homoserine, L-methionine, and downstream products of L-lysine, the downstream products of L-lysine are pentanediamine, 5-aminopentanoic acid, and glutaric acid.
